(19) Europäisches Patentamt
European Patent Office
Office européen des brevets

(11) Numéro de publication: **0 500 446 B1**

# (12) FASCICULE DE BREVET EUROPEEN

(45) Date de publication de fascicule du brevet: **03.05.95**

(51) Int. Cl.$^{6}$: **A61K 7/42**

(21) Numéro de dépôt: **92400419.5**

(22) Date de dépôt: **18.02.92**

(54) **Composition pour la coloration de la peau à base de dérivés indoliques et d'hydroxyacétone.**

(30) Priorité: **22.02.91 FR 9102174**

(43) Date de publication de la demande:
**26.08.92 Bulletin 92/35**

(45) Mention de la délivrance du brevet:
**03.05.95 Bulletin 95/18**

(84) Etats contractants désignés:
**AT BE CH DE DK ES FR GB GR IT LI NL PT SE**

(56) Documents cités:
**EP-A- 0 239 826**
**FR-A- 1 293 290**

(73) Titulaire: **L'OREAL**
**14, Rue Royale**
**F-75008 Paris (FR)**

(72) Inventeur: **Hansenne-Richoux, Isabelle**
**156-158 Rue Legendre**
**F-75017 Paris (FR)**
Inventeur: **Forestier, Serge**
**16 Allée Ferdinand Buisson**
**F-77410 Claye-Souilly (FR)**

(74) Mandataire: **Casalonga, Axel et al**
**BUREAU D.A. CASALONGA - JOSSE**
**Morassistrasse 8**
**D-80469 München (DE)**

EP 0 500 446 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention est relative à l'association de dihydroxyacétone et de dérivés indoliques disubstitués pour conférer à la peau une coloration similaire à la coloration conférée à la peau par un bronzage naturel, ainsi qu'aux procédés et agents de coloration la mettant en oeuvre.

La dihydroxyacétone ou DHA est connue depuis de nombreuses années dans son application à la coloration de la peau.

Il a également été proposé de colorer la peau en utilisant des composés indoliques et en particulier des dérivés du 5,6-dihydroxyindole, ce qui fait l'objet de l'EP-A-239 826.

Ces différentes possibilités de conférer à la peau une coloration présentent cependant chacune des inconvénients.

La montée de la coloration par la DHA, due essentiellement à une réaction de type MAILLARD entre la DHA et les acides aminés de la peau, est lente. Par ailleurs, la coloration obtenue est généralement assez jaune et relativement éloignée de celle d'un bronzage naturel.

D'autre part, la coloration obtenue avec les dérivés indoliques précités, est également éloignée des nuances d'un bronzage naturel et présente, par ailleurs, l'inconvénient de mal résister à l'eau.

Les inventeurs ont découvert, de façon surprenante, qu'en associant la dihydroxyacétone à certains dérivés indoliques disubstitués, il était possible d'obtenir une coloration intense se développant rapidement dans le temps, beaucoup plus proche, par ses couleurs plus chaudes, de la coloration conférée à la peau par un bronzage naturel que les colorations obtenues avec chacun des composés pris isolément, notamment avec la dihydroxyacétone seule. La coloration ainsi obtenue présente, par ailleurs, une très bonne ténacité dans le temps et en particulier au lavage.

Un objet de l'invention est conc constitué par l'association de dihydroxyacétone et d'au moins un dérivé indolique disubstitué, pour conférer à la peau une coloration similaire à celle résultant d'un bronzage naturel.

Un autre objet de l'invention est constitué par des agents de coloration utilisés en cosmétique, destinés à une application topique, contenant les différents composants de l'association selon l'invention.

L'invention a également pour objet un procédé permettant de conférer à la peau une coloration similaire à celle résultant d'un bronzage naturel de la peau, par application de l'association précitée.

D'autres objets de l'invention apparaîtront à la lecture de la description et des exemples qui suivent.

L'association, conforme à l'invention, destinée à conférer à la peau une coloration similaire à la coloration résultant du bronzage naturel, est essentiellement caractérisée par le fait qu'elle comprend :

un composant (A) contenant dans un milieu approprié pour une application topique, de la dihydroxyacétone, et

un composant (B) contenant dans un milieu approprié pour la peau, au moins un colorant indolique disubstitué, répondant à la formule :

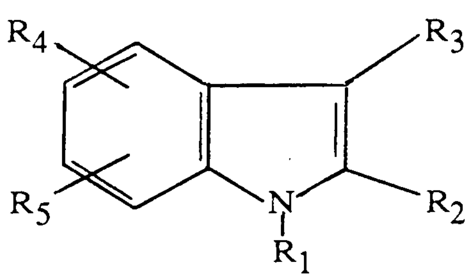

(I)

dans laquelle :

$R_1$, $R_2$ et $R_3$ désignent, indépendamment l'un de l'autre, hydrogène ou un groupement alkyle en $C_1$-$C_4$,

l'un des substituants $R_4$ et $R_5$ désigne alcoxy en $C_1$-$C_4$, et l'autre désigne un groupement hydroxy, les substituants $R_4$ et $R_5$ étant en position ortho ou para l'un par rapport à l'autre, sous réserve que lorsque $R_4$ et $R_5$ sont en positions 5 et 6 du cycle benzénique, $R_4$ en position 5 désigne hydroxy et $R_5$ alcoxy;

ainsi que les sels d'addition de ces composés;

les composants (A) et (B) faisant partie d'une même composition ou étant destinés, soit à être mélangés tout juste avant l'emploi, soit à être appliqués de façon successive ou décalée dans le temps.

Les composés particulièrement préférés, répondant à la formule (I), sont choisis parmi le 4-hydroxy 5-méthoxyindole, le 6-hydroxy 7-méthoxyindole et le 5-hydroxy 6-méthoxyindole.

Comme sels d'addition de ces composés, on peut citer les chlorhydrates, les sulfates et les bromhydrates.

Une première variante de l'invention consiste à utiliser l'association conforme à l'invention, sous forme d'une composition unique, cette composition unique étant, soit préparée et stockée, soit mélangée tout juste avant l'emploi.

Dans ce cas, la dihydroxyacétone est utilisée dans des proportions comprises entre 0,1 et 10% en poids et en particulier entre 1 et 6%, par rapport au poids total de la composition, contenant les composants (A) et (B), et les dérivés indoliques disubstitués sont présents dans des proportions comprises entre 0,1 et 10% en poids, de préférence entre 0,1 et 5% et plus particulièrement entre 0,8 et 2% par rapport au poids total de la composition contenant les composants (A) et (B).

Selon une seconde variante, les composants (A) et (B) sont appliqués, soit de façon successive, soit de façon décalée dans le temps. Dans ce cas, la dihydroxyacétone est présente dans le composant (A) dans des proportions comprises de préférence entre 0,1 et 10% en poids par rapport au poids total du composant (A) et en particulier entre 1 et 6%.

Le ou les dérivés indoliques disubstitués sont présents dans le composant (B) dans des proportions de préférence comprises entre 0,1 et 10% en poids, en particulier entre 0,1 et 5% en poids et de préférence entre 0,8 et 2% en poids par rapport au poids total du composant (B).

Le rapport en poids entre le dérivé indolique disubstitué de formule (I) et la dihydroxyacétone, est généralement supérieur à 0,01.

Le milieu cosmétiquement acceptable approprié pour une application topique, comprend généralement de l'eau, un mélange d'eau et d'un ou plusieurs solvant(s) organique(s), un mélange de solvants organiques ou encore des corps gras, cosmétiquement acceptables.

Ces compositions peuvent éventuellement être pressurisées dans les dispositifs aérosols en présence d'un agent propulseur, éventuellement en présence d'un générateur de mousse.

Les solvants utilisables sont choisis parmi les alcools inférieurs en $C_1$-$C_4$, tels que l'alcool éthylique, l'alcool isopropylique; les monoalcools saturés à longue chaîne ayant 10 à 18 atomes de carbone; les polyols ayant 2 à 8 atomes de carbone parmi lesquels on peut citer le glycérol; les alkylèneglycols comme l'éthylèneglycol, le propylène glycol, le diéthylèneglycol; les éthers de glycols parmi lesquels on peut citer les monoéthylèneglycol monoalkyléthers, les diéthylèneglycol monoalkyléthers, les triéthylèneglycol monoalkyléthers, dans lesquels le groupement alkyle a de préférence 1 à 4 atomes de carbone, tels que par exemple l'éthylèneglycol monoéthyléther, l'éthylèneglycol monobutyléther, le diéthylèneglycol monoéthyléther; les esters acétiques de monoalkyl($C_1$-$C_3$)éthers de l'éthylène glycol, tels que l'acétate de monométhyléther de l'éthylèneglycol et l'acétate de monoéthyléther de l'éthylèneglycol; les esters d'acides gras saturés ayant de préférence 14 à 16 atomes de carbone et d'alcools saturés ayant de 1 à 4 atomes de carbone, parmi lesquels on peut citer le myristate d'isopropyle et le palmitate d'isopropyle.

Parmi les solvants énumérés ci-dessus, on préfère l'alcool éthylique, l'alcool cétylique, le propylèneglycol, l'éthylèneglycol monoéthyléther et l'éthylèneglycol monobutyléther.

Les solvants, lorsqu'ils sont utilisés dans le milieu aqueux, sont présents de préférence dans des proportions comprises entre 0,5 et 75% en poids et en particulier entre 2 et 50% en poids par rapport au poids total de la composition globale ou de chacun des composants (A) et (B).

Comme corps gras utilisables, on peut citer :

- les huiles et cires d'origine minérale, animale ou végétale,
- les huiles de silicone,
- les acides gras,
- les alcools gras, tels que les alcools laurique, cétylique, myristique, stéarique, palmitique, oléique,
- les esters d'acides gras, tels que le monostéarate de glycérol, le monostéarate de polyéthylèneglycol,
- les triglycérides d'acides gras en $C_6$-$C_{18}$.

Le milieu approprié pour une application topique peut être épaissi ou non. Pour l'épaissir, on peut utiliser les agents épaississants ou gélifiants bien connus dans l'état de la technique, tels que la gomme de guar, les hétérobiopolysaccharides, tels que la gomme de xanthane et les scléroglucanes, les dérivés de cellulose, tels que la méthylcellulose, l'hydroxyéthylcellulose, l'hydroxypropylmérhyl cellulose, les sels de sodium de la carboxyméthylcellulose et les polymères d'acide acrylique préférentiellement réticulés.

Lorsque le milieu renferme de l'eau, le pH de la composition unique renfermant les composants (A) et (B) peut varier entre 3 et 10 et il est de préférence compris entre 3 et 7.

Lorsque les composants (A) et (B) sont utilisés séparément, le pH du composant (A) peut varier entre 3 et 10 et il est plus particulièrement compris entre 3 et 7, tandis que le pH du composant (B) peut varier entre 3 et 10 et il est compris plus particulièrement entre 4 et 7.

Les compositions constituées, soit par l'un et/ou l'autre des composants (A) et (B) ou par la composition contenant les deux composants (A) et (B), peuvent être utilisées sous forme de crèmes, de liquides plus ou moins épaissis, de dispersions vésiculaires de lipides amphiphiles ioniques ou non ioniques, d'huiles ou d'aérosols.

Les dispersions vésiculaires de lipides amphiphiles ioniques ou non-ioniques peuvent être préparées selon des procédés connus.

On peut, par exemple, faire gonfler les lipides dans une solution aqueuse pour former des sphérules dispersées dans le milieu aqueux comme décrit dans l'article BANGHAN, STANDISH & WATKINS, J. mol. Biol., 13, 238 (1965) ou dans les brevets FR-2 315 991 et 2 416 008 de la demanderesse.

Ces compositions peuvent également contenir tous autres adjuvants utilisés habituellement dans les compositions cosmétiques pour la peau et plus particulièrement des agents tensio-actifs, de préférence non-ioniques, des agents hydratants, adoucissants, des vitamines, des opacifiants, des parfums ou des agents conservateurs.

Les composants (A) et/ou (B) constituant la composition peuvent également contenir des filtres solaires filtrant le rayonnement UV-B et/ou UV-A.

A titre de filtres solaires, on peut citer les dérivés de l'acide cinnamique, le benzylidènecamphre et ses dérivés, l'acide paraaminobenzoïque et ses dérivés, les dérivés de l'acide salicylique, les dérivés de benzophénone, les homosalates, les dérivés du dibenzoylméthane.

Ces filtres sont présents dans des proportions allant de 0,2 à 10% en poids par rapport au poids du constituant (A) et/ou (B).

Les solvants, corps gras ou adjuvants utilisés dans les compositions de l'invention, ne doivent comporter ni groupements amine primaire, ni groupements oxydants.

L'invention concerne un agent destiné à conférer à la peau une coloration similaire à la coloration résultant d'un bronzage naturel, caractérisé en ce qu'il comprend un premier composant (A) comprenant, dans un milieu approprié pour une application topique, la dihydroxyacétone et un second composant (B) comprenant, dans un milieu approprié pour une application topique, au moins un dérivé indolique disubstitué de formule (I).

Lorsque les composants (A) et (B) sont stockés séparément, l'association conforme à l'invention peut être conditionnée dans un dispositif à plusieurs compartiments encore appelé "kit" ou "nécessaire de coloration de la peau", comportant un premier compartiment contenant le composant (A) renfermant la dihydroxyacétone dans un milieu approprié pour une application topique et un second compartiment contenant le composant (B) renfermant, dans un milieu approprié pour une application topique, au moins un dérivé indolique disubstitué de formule (I).

Une variante de ce dispositif peut consister à l'équiper d'un ensemble distributeur à double compartiment, tel que décrit par exemple par la demanderesse dans la demande de brevet français n° 2 586 913 et comportant deux poches séparées réunies dans un étui souple, les deux poches renfermant, pour l'une d'entre elles, le composant (A) tel que défini ci-dessus, et l'autre poche renfermant le composant (B) tel que défini ci-dessus.

Le procédé de traitement, en vue de conférer à la peau une coloration similaire à la coloration résultant d'un bronzage naturel de celle-ci, est essentiellement caractérisé par le fait que l'on applique le composant (A) et le composant (B) sur la peau, soit au moyen d'une seule composition éventuellement préparée tout juste avant l'emploi, soit en appliquant de façon successive le composant (A) et le composant (B) sur les parties de la peau destinées à être colorées, cette application pouvant éventuellement être décalée dans le temps de quelques minutes à plusieurs heures.

Conformément au procédé de l'invention, le composant (A) peut être appliqué avant ou après l'application du composant (B).

Les exemples suivants sont destinés à illustrer l'invention.

EXEMPLE 1

On prépare la composition suivante :

COMPOSITION (A)

Dans une première étape, on prépare un constituant (A1) comprenant les vésicules.

On fond, en agitant doucement à une température de 95°C, un mélange comportant 3,8 g de lipides non-ioniques, de formule :

$$C_{16}H_{33}\text{-}[OCH_2\text{ - }\underset{\displaystyle CH_2OH}{\underset{|}{C}H}]_n\text{———}OH$$

où n est une valeur statistique moyenne égale à 3,

3,8 g de cholestérol,

0,4 g de sel monosodique du glutamate de stéaroyle vendu sous la dénomination "ACYLGLUTAMATE HS 11" par la Société AJINOMOTO.

On introduit dans le mélange fondu 16 g d'eau portée à 90 °C, contenant un conservateur et l'on mélange pendant environ 5 minutes. A la phase ainsi obtenue, on ajoute 24 g d'eau à 20 °C et on affine le mélange par un passage dans un homogénéiseur haute pression à 500 bars (Rannie).

Dans une seconde étape, on prépare un constituant (A2) comprenant la phase aqueuse de dispersion :

| | | |
|---|---|---|
| - Dihydroxyacétone | | 5,0 g |
| - Paraméthoxycinnamate de 2-éthylhexyle vendu sous la dénomination "ESCALOL 557" par la Société VAN DYK | | 2,0 g |
| - 2-hydroxy 4-méthoxybenzophénone | | 0,5 g |
| - Glycérol | | 2,0 g |
| - Huile de vaseline | | 8,0 g |
| - Huile de silicone | | 5,0 g |
| - Hydroxyéthylcellulose vendue sous la dénomination "NATROSOL PLUS GRADE 330 CS" par la Société AQUALON | | 0,5 g |
| - Conservateurs, parfum | qs | |
| - Eau déminéralisée | qsp | 50,0 g |

On réalise le mélange du constituant (A1) et du constituant (A2) et on homogénéise sous agitation douce.

COMPOSITION (B)

| | | |
|---|---|---|
| - 4-hydroxy 5-méthoxyindole | | 1,0 g |
| - Alcool éthylique | | 43,8 g |
| - Eau déminéralisée | qsp | 100,0 g |

La coloration de la peau peut être obtenue selon les différents modes d'application suivants :

- on applique la composition (A) puis la même quantité en poids de la composition (B) sur la peau;
- on applique la composition (B) puis la même quantité en poids de la composition (A) sur la peau;
- on applique un mélange équipondéral de la composition (A) et de la composition (B) sur la peau.

La coloration obtenue sur une peau non bronzée selon l'un quelconque de ces trois modes d'application, est identique à celle d'un bronzage naturel.

EXEMPLE 2

On prépare les compositions suivantes :

COMPOSITION (A)

Le mode de préparation est identique à celui de l'exemple 1.
Pour le constituant (A1), on utilise :

| | | |
|---|---|---|
| - Lipides non-ioniques de formule : $C_{16}H_{33}-[OCH_2-\underset{\displaystyle CH_2OH}{CH}]_n-OH$ où n est une valeur statistique moyenne égale à 3, | | 3,8 g |
| - sel monosodique du glutamate de stéaroyle vendu sous la dénomination "ACYLGLUTAMATE HS 11" par la Société AJINOMOTO | | 0,4 g |
| - Cholestérol | | 3,8 g |

Pour le constituant (A2), on utilise :

| | | |
|---|---|---|
| - Dihydroxyacétone | | 5,0 g |
| - Glycérol | | 2,0 g |
| - Huile de vaseline | | 8,0 g |
| - Huile de silicone | | 5,0 g |
| - Hydroxyéthylcellulose vendue sous la dénomination "NATROSOL PLUS GRADE 330 CS" par la Société AQUALON | | 0,5 g |
| - Conservateurs, parfum | qs | |
| - Eau déminéralisée | qsp | 100,0 g |

COMPOSITION (B)

| | | |
|---|---|---|
| - 6-hydroxy 7-méthoxyindole | | 1,0 g |
| - Alcool éthylique | | 43,8 g |
| - Eau déminéralisée | qsp | 100,0 g |

La coloration de la peau peut être obtenue selon les différents modes d'application suivants :

- on applique la composition (A) puis la même quantité en poids de la composition (B) sur la peau;
- on applique la composition (B) puis la même quantité en poids de la composition (A) sur la peau;
- on applique un mélange équipondéral de la composition (A) et de la composition (B) sur la peau.

La coloration obtenue sur une peau non bronzée selon l'un quelconque de ces modes d'application, est identique à celle d'un bronzage naturel.

EXEMPLE 3

On prépare les compositions suivantes :

COMPOSITION (A)

La composition (A) est identique à celle de l'exemple 1.

COMPOSITION (B)

| | | | |
|---|---|---|---|
| - 5-hydroxy 6-méthoxyindole | | 1,0 | g |
| - Alcool éthylique | | 49,5 | g |
| - Eau déminéralisée | qsp | 100,0 | g |

La coloration de la peau peut être obtenue selon les différents modes d'application suivants :

- on applique la composition (A) puis la même quantité en poids de la composition (B) sur la peau;
- on applique la composition (B) puis la même quantité en poids de la composition (A) sur la peau;
- on applique un mélange équipondéral de la composition (A) et de la composition (B) sur la peau.

La coloration obtenue sur une peau non bronzée selon l'un quelconque de ces modes d'application, est identique à celle d'un bronzage naturel.

**Revendications**

**1.** Association destinée à conférer à la peau une coloration similaire à la coloration résultant d'un bronzage naturel, caractérisée par le fait qu'elle comprend :

a) un composant (A) contenant dans un milieu approprié pour une application topique, de la dihydroxyacétone; et

b) un composant (B) contenant dans un milieu approprié pour une application topique, au moins un dérivé indolique disubstitué, répondant à la formule :

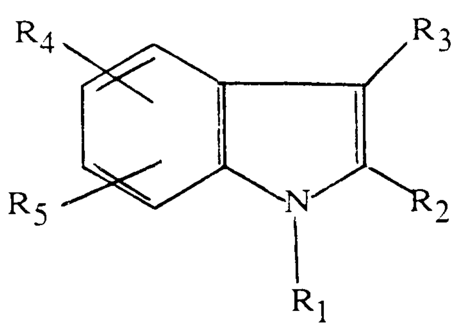

(I)

dans laquelle :

$R_1$, $R_2$ et $R_3$ désignent, indépendamment l'un de l'autre, hydrogène ou un groupement alkyle en $C_1$-$C_4$,

l'un des substituants $R_4$ et $R_5$ désigne alcoxy en $C_1$-$C_4$, et l'autre désigne un groupement hydroxy, les substituants $R_4$ et $R_5$ étant en position ortho ou para l'un par rapport à l'autre, sous réserve que lorsque $R_4$ et $R_5$ sont en positions 5 et 6 du cycle benzénique, $R_4$ en position 5

désigne hydroxy et $R_5$ alcoxy;

ainsi que les sels d'addition de ces composés;

les composants (A) et (B) faisant partie d'une même composition ou étant destinés, soit à être mélangés tout juste avant l'emploi, soit à être appliqués de façon successive ou décalée dans le temps.

**2.** Association selon la revendication 1, caractérisée par le fait que le dérivé indolique disubstitué de formule (I) est choisi parmi le 4-hydroxy 5-méthoxyindole, le 6-hydroxy 7-méthoxyindole et le 5-hydroxy 6-méthoxyindole.

**3.** Association selon la revendication 1 ou 2, caractérisée par le fait que les composants (A) et (B) constituent une seule composition contenant 0,1 à 10% en poids et de préférence 1 à 6% en poids de dihydroxyacétone et 0,1 à 10% en poids, de préférence 0,1 à 5% en poids de dérivé indolique disubstitué de formule (I), ces pourcentages étant exprimés par rapport au poids total de la composition.

**4.** Association selon la revendication 3, caractérisée en ce que la composition comporte 0,8 à 2% en poids du dérivé indolique disubstitué de formule (I), par rapport au poids total de la composition.

**5.** Association selon la revendication 1 ou 2, caractérisée par le fait que les composants (A) et (B) sont séparés, le composant (A) contenant la dihydroxyacétone dans des proportions comprises entre 0,1 et 10% en poids et en particulier entre 1 et 6% en poids par rapport au poids total du composant (A), le composant (B) contenant le dérivé indolique disubstitué de formule (I) dans des proportions comprises entre 0,1 et 10% en poids et de préférence entre 0,1 et 5% en poids par rapport au poids total du composant (B).

**6.** Association selon la revendication 5, caractérisée par le fait que le composant (B) contient entre 0,8 et 2% en poids du dérivé indolique disubstitué de formule (I).

**7.** Association selon l'une quelconque des revendications 1 à 6, caractérisée par le fait que le rapport en poids entre le dérivé indolique disubstitué de formule (I) et la dihydroxyacétone, est supérieur à 0,01.

**8.** Association selon l'une quelconqe des revendications 1 à 7, caractérisée par le fait que le milieu approprié pour l'application topique est un milieu comprenant de l'eau, un mélange d'eau et d'un ou plusieurs solvant(s) organique(s), un mélange de solvants organiques ou des corps gras, ces derniers et les solvants organiques étant cosmétiquement acceptables.

**9.** Association selon l'une quelconque des revendications 1 à 8, caractérisée par le fait que les solvants sont choisis parmi les alcools inférieurs en $C_1$-$C_4$, les monoalcools saturés à longue chaine ayant 10 à 18 atomes de carbone, les polyols ayant 2 à 8 atomes de carbone, les alylèneglycols, les éthers de glycols, les esters acétiques de monoalkyl($C_1$-$C_3$)éthers de l'éthylèneglycol, les esters d'acides gras saturés ayant 14 à 16 atones de carbone et d'alcools saturés ayant 1 à 4 atomes de carbone.

**10.** Association selon l'une quelconque des revendications 1 à 9, caractérisée par le fait que les solvants sont choisis parmi l'alcool éthylique, l'alcool cétylique, le propylèneglycol, l'éthylèneglycol monoéthyléther et l'éthylèneglycol monobutyléther.

**11.** Association selon l'une quelconque des revendications 1 à 10, caractérisée par le fait que les corps gras sont choisis parmi les huiles minérales, végétales ou animales, les acides gras, les alcools gras , les huiles de silicone, les esters d'acides gras, les triglycérides d'acides gras.

**12.** Association selon l'une quelconque des revendications 1 à 11, caractérisée par le fait que les milieux appropriés pour une application topique sont épaissis.

**13.** Association selon l'une quelconque des revendications 1 à 12, caractérisée par le fait que le pH du composant (A) est compris entre 3 et 10 et de préférence entre 3 et 7 et que le pH du composant (B) est compris entre 3 et 10 et de préférence entre 4 et 7.

**14.** Association selon l'une quelconque des revendications 1 à 13, caractérisée par le fait que les composants (A) et (B) se présentent sous forme de crèmes, de liquides plus ou moins épaissis, de dispersions vésiculaires de lipides amphiphiles ioniques ou nonioniques, de compositions aérosols ou d'huiles.

**15.** Association selon l'une quelconque des revendications 1 à 14, caractérisée par le fait que l'un au moins des constituants (A) et (B) contient au moins un filtre solaire filtrant le rayonrrement UV-A et/ou UV-B.

**16.** Association selon l'une quelconque des revendications 1 à 15, caractérisée par le fait que les composants (A) et (B) contiennent en plus des agents tensio-actifs, des agents hydratants, adoucissants, opacifiants, des vitamines, des parfums ou des agents conservateurs.

**17.** Agent destiné à conférer à la peau une coloration similaire à la coloration résultant d'un bronzage naturel, caractérisé par le fait qu'il comprend un premier composant (A) comprenant dans un milieu approprié pour une application topique, la dihydroxyacétone et un second composant (B) comprenant dans un milieu approprié pour une application topique, au moins un dérivé indolique disubstitué de formule (I) défini dans la revendication 1.

**18.** Dispositif à plusieurs compartiments ou "kit" ou "nécessaire", pour conférer à la peau une coloration similaire à la coloration résultant d'un bronzage naturel, caractérisé par le fait qu'il est constitué par un dispositif à deux poches séparées réunies dans un étui souple, la première poche renfermant le composant (A) tel que défini dans la revendication 1, et la seconde poche renfermant le composant (B) tel que défini dans la revendication 1.

**19.** Procédé de coloration de la peau afin de lui conférer une coloration similaire à celle d'un bronzage naturel, caractérisé par le fait que l'on applique sur la peau l'association telle que définie dans l'une quelconque des revendications 1 à 16.

## Claims

**1.** Combination intended to impart to the skin a colour similar to the colour resulting from natural tanning, characterised in that it comprises:

a) a component (A) containing dihydroxyacetone in a medium suitable for a topical application; and

b) a component (B) containing, in a medium suitable for a topical application, at least one disubstituted indole derivative corresponding to the formula:

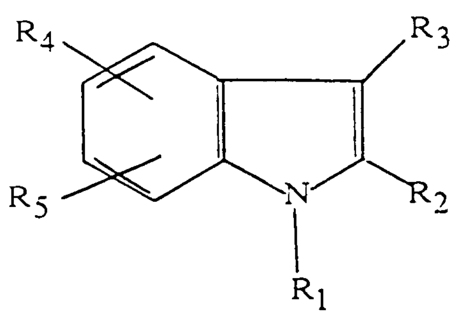

(I)

in which:

$R_1$, $R_2$ and $R_3$ denote, independently of each other, hydrogen or a $C_1$-$C_4$ alkyl group,

one of the substituents $R_4$ and $R_5$ denotes $C_1$-$C_4$ alkoxy and the other denotes a hydroxyl group, the substituents $R_4$ and $R_5$ being in an ortho or para position relative to each other, provided that when $R_4$ and $R_5$ are in positions 5 and 6 of the benzene ring, $R_4$ in position 5 denotes hydroxyl and $R_5$ alkoxy;

as well as the addition salts of these compounds;

the components (A) and (B) forming part of the same composition or being intended either to be mixed just before use or to be applied successively or with a lag in time.

2. Combination according to Claim 1, characterised in that the disubstituted indole derivative of formula (I) is chosen from 4-hydroxy-5-methoxyindole, 6-hydroxy-7-methoxyindole and 5-hydroxy-6-methoxyindole.

3. Combination according to Claim 1 or 2, characterised in that the components (A) and (B) constitute a single composition containing 0.1 to 10 % by weight and preferably 1 to 6 % by weight of dihydroxyacetone and 0.1 to 10 % by weight, preferably 0.1 to 5 % by weight of disubstituted indole derivative of formula (I), these percentages being expressed in relation to the total weight of the composition.

4. Combination according to Claim 3, characterised in that the composition comprises 0.8 to 2 % by weight of the disubstituted indole derivative of formula (I), relative to the total weight of the composition.

5. Combination according to Claim 1 or 2, characterised in that the components (A) and (B) are separated, the component (A) containing dihydroxyacetone in proportions of between 0.1 and 10 % by weight and in particular between 1 and 6 % by weight relative to the total weight of the component (A), the component (B) containing the disubstituted indole derivative of formula (I) in proportions between 0.1 and 10 % by weight and preferably between 0.1 and 5 % by weight relative to the total weight of the component (B).

6. Combination according to Claim 5, characterised in that the component (B) contains between 0.8 and 2 % by weight of the disubstituted indole derivative of formula (I).

7. Combination according to any one of Claims 1 to 6, characterised in that the weight ratio of the disubstituted indole derivative of formula (I) to dihydroxyacetone is higher than 0.01.

8. Combination according to any one of Claims 1 to 7, characterised in that the medium suitable for the topical application is a medium comprising water, or a mixture of water and of one or more organic solvent(s), a mixture of organic solvents or of fatty substances, the latter and the organic solvents being cosmetically acceptable.

9. Combination according to any one of Claims 1 to 8, characterised in that the solvents are chosen from $C_1$-$C_4$ lower alcohols, long-chain saturated monoalcohols containing 10 to 18 carbon atoms, polyols containing 2 to 8 carbon atoms, alkylene glycols, glycol ethers, acetic esters of ethylene glycol $C_1$-$C_3$ monoalkyl ethers, and esters of saturated fatty acids containing 14 to 16 carbon atoms and of saturated alcohols containing 1 to 4 carbon atoms.

10. Combination according to any one of Claims 1 to 9, characterised in that the solvents are chosen from ethyl alcohol, cetyl alcohol, propylene glycol, ethylene glycol monoethyl ether and ethylene glycol monobutyl ether.

11. Combination according to any one of Claims 1 to 10, characterised in that the fatty substances are chosen from mineral, vegetable or animal oils, fatty acids, fatty alcohols, silicone oils, fatty acid esters and triglycerides of fatty acids.

12. Combination according to any one of Claims 1 to 11, characterised in that the media suitable for a topical application are thickened.

13. Combination according to any one of Claims 1 to 12, characterised in that the pH of the component (A) is between 3 and 10 and preferably between 3 and 7 and that the pH of the component (B) is between 3 and 10 and preferably between 4 and 7.

14. Combination according to any one of Claims 1 to 13, characterised in that the components (A) and (B) are in the form of creams, of more or less thickened liquids, of vesicular dispersions of ionic or nonionic amphiphilic lipids, of aerosol compositions or of oils.

15. Combination according to any one of Claims 1 to 14, characterised in that at least one of the constituents (A) and (B) contains at least one sunscreen filtering out UV-A and/or UV-B radiation.

**16.** Combination according to any one of Claims 1 to 15, characterised in that the components (A) and (B) additionally contain surface-active agents, hydrating agents, emollients, opacifiers, vitamins, perfumes or preserving agents.

**17.** Agent intended to impart to the skin a colour similar to the colour resulting from natural tanning, characterised in that it comprises a first component (A) containing dihydroxyacetone in a medium suitable for a topical application and a second component (B) containing, in a medium suitable for a topical application, at least one disubstituted indole derivative of formula (I) as defined in Claim 1.

**18.** Multicompartment device or kit or outfit for imparting to the skin a colour similar to the colour resulting from natural tanning, characterised in that it consists of a device with two separate pouches placed together in a flexible case, the first pouch containing the component (A) as defined in Claim 1, and the second pouch containing the component (B) as defined in Claim 1.

**19.** Process for colouring the skin in order to impart to it a colour similar to that of a natural tan, characterised in that the combination as defined in any one of Claims 1 to 16 is applied to the skin.

**Patentansprüche**

**1.** Mischung zur Übertragung einer Färbung auf die Haut, welche der Färbung aus einer natürlichen Bräunung ähnlich ist,
dadurch **gekennzeichnet**, daß
sie umfaßt:
a) einen Bestandteil (A), enthaltend in einem zur topischen Aufbringung geeigneten Milieu Dihydroxyaceton, sowie
b) einen Bestandteil (B), der in einem zur topischen Aufbringung geeigneten Milieu mindestens einen disubstituierten Indol-Farbstoff der Formel:

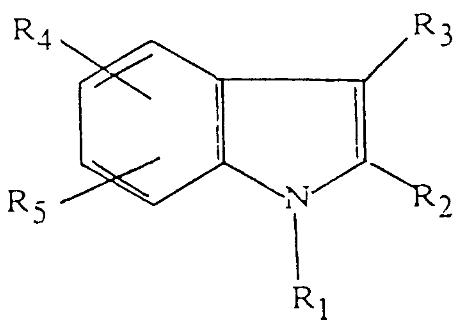

(I)

worin:
$R_1$, $R_2$ und $R_3$, unabhängig voneinander, Wasserstoff oder eine $C_{1-4}$-Alkylgruppe bedeuten,
einer der Substituenten $R_4$ und $R_5$ einen $C_{1-4}$-Alkoxyrest und der andere einen Hydroxyrest bedeuten, wobei sich die Substituenten $R_4$ und $R_5$ in ortho- oder para-Position zueinander befinden, mit der Maßgabe, daß, wenn $R_4$ und $R_5$ in den Positionen 5 und 6 des Benzolrings vorliegen, $R_4$ in Position 5 einen Hydroxyrest und $R_5$ einen Alkoxyrest darstellen,
sowie die Additionsalze dieser Verbindungen enthält,
wobei die Bestandteile (A) und (B) Teile derselben Zusammenstzung darstellen oder dazu bestimmt sind, entweder kurz vor Gebrauch vermischt oder nacheinander oder nach einem Zeitablauf aufgetragen zu werden.

**2.** Mischung gemäß Anspruch 1,
dadurch **gekennzeichnet**, daß
das disubstituierte Indolderivat der Formel (I) aus 4-Hydroxy-5-methoxyindol, 6-Hydroxy-7-methoxyindol und 5-Hydroxy-6-methoxyindol ausgewählt ist.

**3.** Mischung gemäß Anspruch 1 oder 2,
dadurch **gekennzeichnet**, daß

die Bestandteile (A) und (B) eine einzige Zusammensetzung darstellen, die 0,1 bis 10 und vorzugsweise 1 bis 6 Gew.% Dihydroxyaceton und 0,1 bis 10 und vorzugsweise 0,1 bis 5 Gew.% disubstituiertes Indolderivat der Formel (I) enthält, wobei diese Prozentsätze auf das Gesamtgewicht der Zusammensetzung bezogen sind.

**4.** Mischung gemäß Anspruch 3,
dadurch **gekennzeichnet**, daß
die Zusammensetzung 0,8 bis 2 Gew.% disubstituiertes Indolderivat der Formel (I) enthält, bezogen auf Gesamtgewicht der Zusammensetzung.

**5.** Mischung gemäß Anspruch 1 oder 2,
dadurch **gekennzeichnet**, daß
die Bestandteile (A) und (B) getrennt voneinander vorliegen, wobei der Bestandteil (A) das Dihydroxyaceton in Mengenanteilen von 0,1 bis 10 und insbesondere von 1 bis 6 Gew.%, bezogen auf Gesamtgewicht des Bestandteils (A) und der Bestandteil (B) das disubstituierte Indolderivat der Formel (I) in Mengenanteilen von 0,1 bis 10 und vorzugsweise von 0,1 bis 5 Gew.%, bezogen auf Gesamtgewicht des Bestandteils (B), enthalten.

**6.** Mischung gemäß Ansprrpuch 5,
dadurch **gekennzeichnet**, daß
der Bestandteil (B) 0,8 bis 2 Gew.% disubstituiertes Indolderivat der Formel (I) enthält.

**7.** Mischung gemäß jedem der Ansprüche 1 bis 6,
dadurch **gekennzeichnet**, daß
das Gewichtsverhältnis zwischen dem disubstituierten Indolderivat der Formel (I) und dem Dihydroxyaceton oberhalb 0,01 liegt.

**8.** Mischung gemäß jedem der Ansprüche 1 bis 7,
dadurch **gekennzeichnet**, daß
das zur topischen Aufbringung geeignete Milieu ein Milieu ist, das Wasser, eine Mischung aus Wasser und einem oder mehreren organischen Lösungsmittel(n), eine Mischung aus organischen Lösungsmitteln oder Fettkörper umfaßt, wobei die letzteren und die organischen Lösungsmittel kosmetisch zulässige Produkte sind.

**9.** Mischung gemäß jedem der Ansprüche 1 bis 8,
dadurch **gekennzeichnet**, daß
die Lösungsmittel aus $C_{1-4}$-Niedrigalkoholen, gesättigten Monoalkoholen mit einer langen Kette von 10 bis 18 Kohlenstoffatomen, aus Polyolen mit 2 bis 8 Kohlenstoffatomen, Alkylenglycolen, Glycolethern, Essigsäureestern vom $C_{1-3}$-Monoalkylethern von Ethylenglycol und aus Estern gesättigter Fettsäuren mit 14 bis 16 Kohlenstoffatomen und gesättigter Alkohole mit 1 bis 4 Kohlenstoffatomen ausgewählt sind.

**10.** Mischung gemäß einem jeden der Ansprüche 1 bis 9,
dadurch **gekennzeichnet**, daß
die Lösungsmittel aus Ethylalkohol, Cetylalkohol, Propylenglycol, Ethylenglycolmonoethylether und Ethylenglycolmonobutylether ausgewählt sind.

**11.** Mischung gemäß jedem der Ansprüche 1 bis 10,
dadurch **gekennzeichnet**, daß
die Fettkörper aus mineralischen, pflanzlichen oder tierischen Ölen, Fettsäuren, Fettalkoholen, Silikonölen, Fettsäureestern und Triglyceriden von Fettsäuren ausgewählt sind.

**12.** Mischung gemäß jedem der Ansprüche 1 bis 11,
dadurch **gekennzeichnet**, daß
die für eine topische Aufbringung geeigneten Medien verdickt sind.

**13.** Mischung gemäß jedem der Ansprüche 1 bis 12,
dadurch **gekennzeichnet**, daß

der pH-Wert des Bestandteils (A) 3 bis 10 und vorzugsweise 3 bis 7 und der pH-Wert des Bestandteils (B) 3 bis 10 und vorzugsweise 4 bis 7 betragen.

**14.** Mischung gemäß jedem der Ansprüche 1 bis 13,
dadurch **gekennzeichnet**, daß
die Bestandteile (A) und (B) in Form von Creme-Produkten, mehr oder weniger verdickten Flüssigkeiten, bläschenartigen Dispersionen amphiphiler ionischer oder nicht-ionischer Lipide, Aerosol- oder Öl-Zusammensetzungen vorliegen.

**15.** Mischung gemäß jedem der Ansprüche 1 bis 14,
dadurch **gekennzeichnet**, daß
mindestens einer der Bestandteile (A) und (B) mindestens einen Sonnenfilterstoff enthält, der die UV-A- und/oder UV-B-Strahlung filtert.

**16.** Mischung gemäß jedem der Ansprüche 1 bis 15,
dadurch **gekennzeichnet**, daß
die Bestandteile (A) und (B) zusätzlich oberflächenaktive Mittel, hydratisierende Mittel, Weichmacher, opak machende Mittel, Vitamine, Parfüm-Produkte oder Konservierungsmittel enthalten.

**17.** Mittel zur Übertragung einer Färbung auf die Haut, welche der Färbung aus einer natürlichen Bräunung ähnlich ist,
dadurch **gekennzeichnet**, daß
es einen ersten Bestandteil (A), enthaltend in einem für eine topische Aufbringung geeigneten Milieu das Dihydroxyaceton, sowie einen zweiten Bestandteil (B) umfaßt, der in einem für eine topische Aufbringung geeigneten Milieu mindestens ein in Anspruch 1 definiertes disubstituiertes Indolderivat der Formel (I) aufweist.

**18.** Vorrichtung aus mehreren Teilen oder "Kit" oder "Necessaire" zur Übertragung einer Färbung auf die Haut, welche der Färbung aus einer natürlichen Bräunung ähnlich ist,
dadurch **gekennzeichnet**, daß
sie aus einer Vorrichtung mit zwei getrennten Taschen zusammengesetzt sind, welche in einem biegsamen Etui zusammengefaßt sind, wobei die erste Tasche den in Anspruch 1 definierten Bestandteil (A) und die zweite Tasche den in Anspruch 1 definierten Bestandteil (B) umfassen.

**19.** Verfahren zur Färbung der Haut zur Übertragung einer der natürlichen Bräunung ähnlichen Färbung,
dadurch **gekennzeichnet**, daß
man auf die Haut die in jedem der Ansprüche 1 bis 16 definierte Mischung aufträgt.